Europäisches Patentamt

European Patent Office

Office européen des brevets

(19)

(11) Publication number: **0 157 058**
A2

# (12) EUROPEAN PATENT APPLICATION

(21) Application number: **84402382.0**

(22) Date of filing: **22.11.84**

(51) Int. Cl.⁴: **A 61 H  7/00,** A 61 H  39/06, A 61 F  7/00

(30) Priority: **19.12.83  FR 8320273**

(43) Date of publication of application: **09.10.85**
**Bulletin 85/41**

(84) Designated Contracting States: **BE CH DE GB IT LI LU NL**

(71) Applicant: **Bontemps, Raymond, 5, rue Edouard Detaille, F-75017 Paris (FR)**

(72) Inventor: **Bontemps, Raymond, 5, rue Edouard Detaille, F-75017 Paris (FR)**

(54) **Portable device for skin massage by cold.**

(57)   This invention is about a portable cold conservative for skin massage because of the vaso dilatation effect that follows the vasoconstrictor effect of cold.

According to fig. I, the invention is characterized by a small ball that contains glycerine and pure alcohol, preserved at −20 °C. This ball is closed by means of a rubber or plastic teat that is both congelation indicator and application device.

The neck of this ball is protected by an isotherm collarette to make it easy to use.

The invention is used in cosmetology and every treatment by cold.

0157058

*The invention is about a non metallic container, preferently made of glass, ball-shaped with a neck to take it easy . This closed container contains either glycerine and alcohol or pure alcohol and can be stocked at -20°C in a congelator .*

*This device can keep the cold for skin treatment by cold . This device can also be used for a local anesthetization.*

*It was noticed that a cold device ( -10°C to -20°C ) involves a local vasoconstrictor effect if it is applied on the skin, and so impurities of the pores are eliminated . Local warm up follows because of activation of blood circulation the stronger vasoconstriction was, the intenser the vasodilatation was .*

*The alternation vasoconstriction / vasodilatation makes the skin softer and permit to the skin to receive any cosmetic product . For the last few years, with treatments using fresh cells , we have been using always more fregmently foetal substances that have to be conserved at -80°C in a congelator to keep their properties and that are used by applications at -10 or -20°C .*

*It was so noticed that application of these congelated substances can provokes a vaso constrictor effect that involves impurities elimination . Secondly , when warm up , the skin can better absorb the cosmetic product made of fresh cells applied on the skin .*

*From these constatations , it was imagined a device that could provoke a skin vaso constriction in order to ameliorate the absorption of the substance during the vasodilatation .*

Such a device could also make the skin softer by means of a massage. Effectively , in cosmetology , it was used until now several ways with a skin cleaning ( with a soap ) , then a dry cleaning and manual massages . These operations was made to make the skin soft and to ameliorate the absorption of the substance for the treatment .

However , these operations had many drawbacks like :

- The use of chemical products that could spoil the skin if treatments are frequents.

- A long treatment that needs several hours and that could only be in special rooms where there is necessary instrumentation.

- An expensive cost because of use expensive chemical products .

It is to avoid these drawbacks that it is there proposed a portable device for skin massage by cold .

This portable device is characterized in that it is constitued of a small ball containing a few cubic centimeters of either glycerine and alcohol or pure alcohol , this ball is closed by means of a teat that is on the upper part of the ball neck , the lower shank is linked to the teat , the neck of the ball is rounded of rubbon or any isotherm substance to take the device easily , this device is conserved in a congelator at -20°C .

This invention has many advantages and particularly :

- It is easy to use by any one that wish receive a skin local massage, anywhere, without any chemical product .

- It is cheap to use.

- The possibility to use this device for local anesthetization in order to avoid any anesthetic.

- It is easy to apply on any part of the body, even the more difficult to treat.

This invention will better understanden by means of drawings that are presented as examples.

- Fig I shows according to a characteristic of the invention the device by a cross-section view .

- Fig. 2 shows according to another characteristic of the invention, the same device with a screwn teat by a cross-section view.

- Fig. 3 shows the teat and the screwn stopper to maintain this teat full of cold liquid , whatever position has the device during operating.

Seeing Fig.I and according to an important characteristic of the invention , the device is shown according to a sectional elevation. This device is constituted by a 50 cm$^3$ ball that contains either alcohol and glycerine or pure alcohol (2). The ball (I) has a round lower part (13) to make easy the skin application, the upper part has a neck (7) in order to take the device. To take it easily , the neck (7) has a rubber collarette (3) or made of any isotherm material.

The ball (I) is closed at its upper part by a rubber teat (4).
At the level (5), this teat is pear-shaped whose end is a hollow
channel (8) with a pointed part at the level (6). The teat is
placed in order to use the ferrule (6) as the local massage operator.

According to another characteristic of the invention,

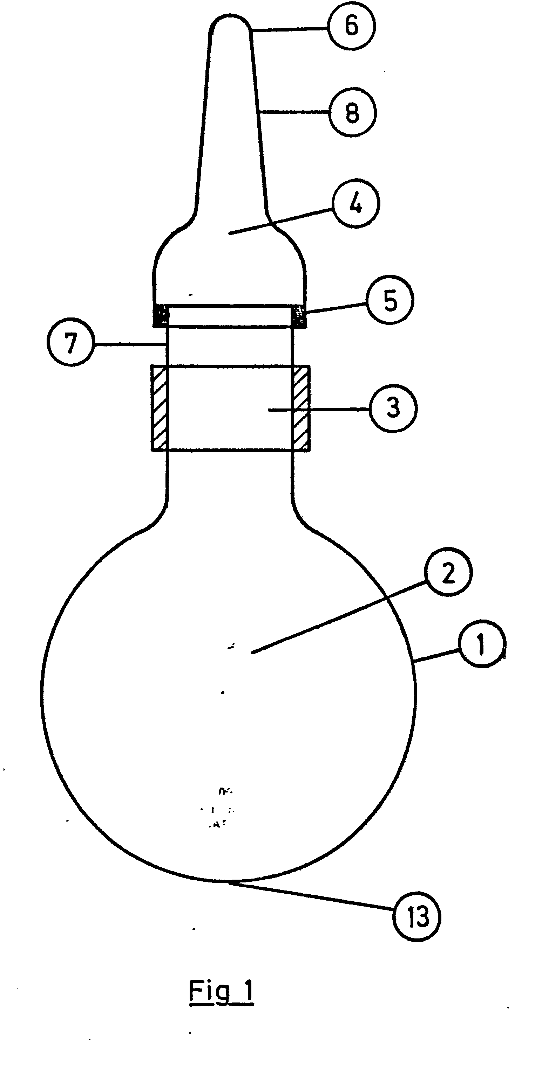

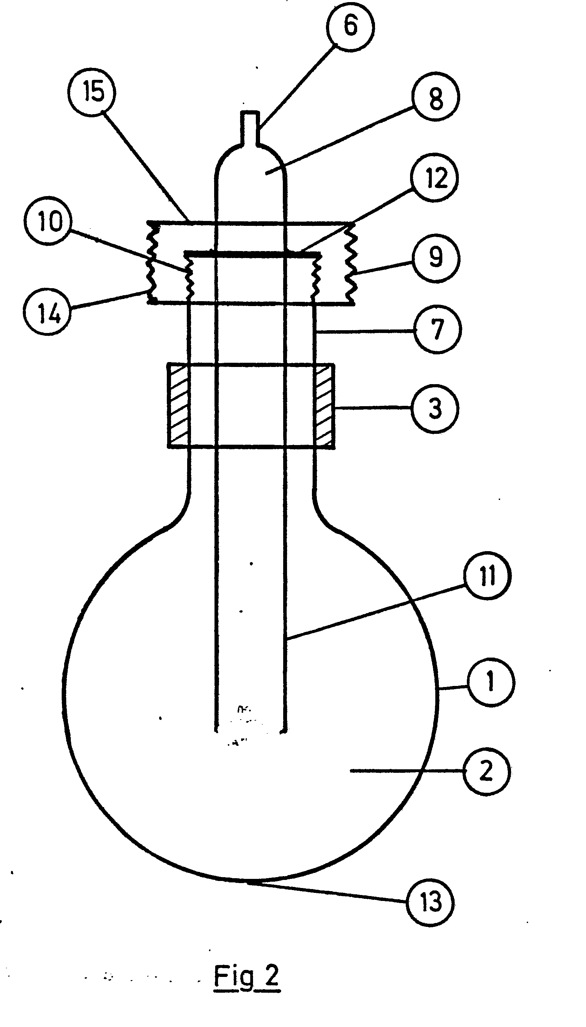

on Fig. 2 is shown the same device with a screw stopper (14) and a
teat (6) full of cold liquid whatever the position of the device
during operating.

The device is composed of a glass ball (5) whose
lower part is round (13) and the upper part has a neck (7) with
a glass moulded. (10) The ball (I) is fulled with either alcohol and
glycerine or with pure alcohol (2) .
The neck of the ball (7) is closed by a screwn plastic stopper (14)
whose threading (9) is according to the ball neck (7) threading (10).
The plastic stopper (14) has, at its upper part , a hole (15) for
the teat (8) . This teat has a ferrule (6) used as the massage
component and its lower part is a hollow shank (11) that goes in
the liquid (2) of the ball (I).
The teat (8) and the hollow shank (11) are made of the same
material ( plastic or rubber ) constitutes a whole with a collarette
(12) to keep the plastic screwn stopper tight .

To take the device easily, the neck of the ball (7)
has a rubber collarette (3) or made of any isotherm plastic .

On Fig. 3 are shown the teat (8) components adapted
to the device describe on Fig. 2. The closed ferrule (6) placed
on the upper part of the teat (8) has a tight joint (12) and a
hollow shank (11) .

The whole is moulded in a plastic material that has physical characteristic of rubber . This teat and its components goes into the hole (15) on the threaded stopper (14) to make it tight .

Use :

The ball (I) is full of either alcohol and glycerine or pure alcohol until the upper level of the neck (7) . The ball receives the teat (8) composed of the tight-joint (12) and the hollow shank (11) that goes into the liquid (2) . The whole becomes tight with the threaded stopper (12) that can be screwn on the threading (10). When these operations are finished , the air of the teat (8) is exhausted and is replaced by the liquid of the ball , by pressuring or setting the device up .
Then the whole is maintained a few hours at -20°C in a congelator .
For a massage, the ball is taken with the collarette (3) and its round lower part (3) is applied on the skin with a low and uncreasing massage. For a local massage, the end (6) of the teat (8) is used that, whatever the position of the device, stays full of the liquid at -10°C or -20°C

Claim 1 - Portable device for skin massage by cold characterized
in that it is constitued of a glass ball with a round lower
part that contains a few cubic centimeters either of
glycerine and alcohol or pure alcohol , a neck that can
receive a threading and a rubber collarette, a closing
device composed of a teat that can be directly adapted or
by means of a threading stopper , the teat is closed by a
soft ferrule that realizes the massage , the lower part of
the teat has a hollow shank that goes into the liquid , the
whole is maintained at -20°C .

Claim 2 - Device according to claim 1 characterized in that the
way to realize the skin massage is a glass ball (1) with
a bottom (13) , that contains a liquid made of glycerine and
alcohol or of pure alcohol that is maintained at -10 or -20°C
in any congelator .

Claim 3 - Device according to claim 1 characterized in that the
way to close the ball is a close teat (8) with a tight-joint
(12) and a closed ferrule (6) for local massage .

Claim 4 - Device according to claim 1 characterized in that the
ball is closed by means of the teat (8) or by using a threaded
stopper (12) whose threading (9) is adapted to the moulded
threading (10) on the top of the neck (7) of the ball .

_Claim 5 -_   _Device according to claim 1 characterized in that_
     _the way to maintain the teat full of cold liquid is a_
     _hollow shank (11) at the end of the teat (8) and whose_
     _a part is always in the liquid (2) of the ball ._

_Claim 6 -_   _Device according to claim 1 characterized in that_
     _the way to take this device is a rubber collarette (3)_
     _or made of an isotherm material , round the neck (7) of_
     _the ball (1) ._

_Claim 7 -_   _Device according to claim 1 characterized in that_
     _the way to get the local massage is a pencil (6) that is_
     _the end of the upper part of the teat (8) always full of_
     _cold liquid ._

1/2

0157058

Fig 1

4|2

0157058

Fig 2